# EUROPÄISCHE PATENTANMELDUNG

(11) **EP 2 853 248 A1**
(43) Veröffentlichungstag der Anmeldung: **01.04.2015**
(21) Anmeldenummer: 14181044.0
(22) Anmeldetag: 14.08.2014
(51) Int. Cl.: A61F 13/12, A61F 13/00

(54) **Augentupfer**

(30) Priorität: 30.09.2013 CH 16642013
(71) Anmelder: Joker AG, 3210 Kerzers/FR (CH)
(72) Erfinder: Flury, Meinrad, 3210 Kerzers (CH); Lee, Bejanmin Tak Kwong, Shatin, N. T. (HK); Chan, Wing Shong Thierry, Lam Tin, Kowloon (HK); Wong, Chun Ho, Shatin, NT (HK); Gan, Yi Ming, Tai Po, NT (HK)
(74) Vertreter: Schneider Feldmann AG Patent- und Markenanwälte

(57) **Zusammenfassung**

Bei einem Augentupfer zur Reinigung von Wirbeltieraugen, beispielsweise von Hundeaugen, ist es erfindungsgemäss vorgesehen, dass der Augentupfer aus einer gelartige Knetmasse besteht, wobei die gelartige Knetmasse aus einem reversibel gebundenen, 3-dimensionalen Netzwerk aus mindestens einem natürlichen oder synthetischen Polymer, mindestens einem Vernetzer und Glycerin zusammengehalten ist.

## Beschreibung

### TECHNISCHES GEBIET

Die Erfindung betrifft einen Augentupfer zur Reinigung von Wirbeltieraugen, beispielsweise von Hundeaugen.

### STAND DER TECHNIK

Die Augen von Haustieren sind äusserst sensitive Bereiche und anfällig gegenüber Staub, losem Haar oder anderen Fremdkörpern, welche mit dem Wind oder auf anderen Wegen in die Augen gelangen können. Insbesondere Hundebesitzer reinigen die Augen ihrer Tiere oft mit einem mit lauwarmem Wasser benetzten Tuch, um solche Anreicherungen von Fremdkörpern aus den Augenwinkeln im Bereich der Tränendrüsen zu entfernen. Die Fremdkörper und damit eingetragene Bakterien können sonst zu Entzündungen, Schmerzen und sonstigen Beschwerden führen, welche zudem eine erhöhte Tränenproduktion bewirken. Die erhöhte Tränenproduktion wiederum führt oft zu unerwünschten und unästhetischen Tränenflecken, insbesondere auf z.B. einem hellem Fell im Augenbereiche des Hundes.

Für Menschen sind zur Reinigung und Pflege von Augen sterile, feuchte und nicht fasernde Reinigungstücher bekannt. Diese sind meist einzeln und steril verpackt, und können mit Pflanzenextrakten getränkt sein, welche beruhigend, abschwellend und antiseptisch auf die empfindliche Haut der Augenpartie wirken. Ebenfalls bekannt sind Reinigungsgels, welche auf ein Papiertuch oder auf die Reinigungstücher gegeben werden. Mit der Verwendung von Tüchern besteht jedoch eine Verletzungsgefahr bei Berührung der Horn- oder Lederhaut des Auges.

Zur Reinigung von Augen werden auch Augenbäder eingesetzt. Diese Bäder spülen die Augen und habe eine erfrischende Wirkung auf trockene und gereizte Augen. Sie werden häufig von Allergikern eingesetzt, um Staub oder Pollen aus den Augen auszuspülen, sind aber für Haustiere aufgrund der umständlichen Anwendung ungeeignet.

Aus WO 02/055642 ist ein gelförmiges Reinigungsmittel resp. eine gelförmige Knetmasse aus natürlichen Polygalactomananen mit einem Anteil von 75 bis 95 Gew.-% Wasser zur fusselfreien Entfernung von Feststoffpartikeln bekannt. Eine Weiterentwicklung dieser gelförmigen Knetmasse zur Reinigung von Haustierpfoten wird in WO2013/056972 beschrieben. Dieses Haustierpfotenreinigungsmittel enthält zusätzlich ein desinfizierendes Lösungsmittel aus der Gruppe der gesättigten, einwertigen Alkohole und Lanolin.

### DARSTELLUNG DER ERFINDUNG

Eine Aufgabe der Erfindung ist es, einen Augentupfer zur Reinigung von Wirbeltieraugen, beispielsweise von Hundeaugen, anzugeben, mit welchem Anreicherungen von Schmutz und ggf. Tränenflecken sanft und einfach aus den Augen und ggf. vom umliegenden Fell entfernt werden können. Eine Schädigung der Horn- oder Lederhaut des Auges durch allfällige Berührung mit dem Augentupfer soll dabei vermieden werden. Gemäss einer weiteren Aufgabe soll der Augentupfer derart beschaffen sein, dass er einfach mit entzündungshemmend oder beruhigend wirkenden Substanzen versehen werden kann, um den Komfort und das Wohlbefinden des Haustieres zu verbessern. Auch soll er bei der Anwendung an Haustieren dieses wenig belasten. Eine weitere Aufgabe besteht darin, dass der Augentupfer mehrmals wieder verwendbar ist. Ebenso soll der Augentupfer mit antiseptischen Substanzen angereichert werden können.

Im Sinne der Erfindung wird unter dem Begriff Auge insbesondere der von Aussen her zugänglichen Bereich der Augenlider, die Wimpern und die Nickhaut verstanden, wobei auch die Hornhaut und die Lederhaut beim Kontakt mit dem Augentupfer keinen schaden nehmen würde.

Unter gelartiger Knetmasse wird eine hochviskose Masse verstanden, die leicht deformierbar ist und bei welcher durch einige Knetbewegungen Verunreinigungen an der Oberfläche ins Innere der Masse einknetbar sind. Die gelartige Knetmasse lässt sich strecken und zusammendrücken aber auch reissen und wieder zusammenfügen.

In einer Ausführungsform besteht der Augentupfer zur Reinigung von Wirbeltieraugen, beispielsweise von Hundeaugen, aus einer gelartige Knetmasse. Die gelartige Knetmasse ist aus einem reversibel gebundenen, 3-dimensionalen Netzwerk aus mindestens einem natürlichen oder synthetischen Polymer, mindestens einem Vernetzer und Glycerin zusammengehalten. Ein solcher Augentupfer ist sehr flexible und weich und ohne Schädigung des Auges anwendbar. Die gelartige Knetmasse kann einfach mit entzündungshemmend, beruhigend und/oder antiseptisch wirkenden Substanzen versehen werden, welche dazu im 3-dimensionalen Netzwerk eingebunden oder eingebettet sind und dosiert abgegeben werden können.

Das reversibel gebundene, 3-dimensionale Netzwerk der gelartigen Masse wird über verschiedene chemische Gleichgewichte zwischen den Komponenten (Polymer, Vernetzer und Glycerin) zusammengehalten, welche es erlauben, dass die Verbindungen zwischen dem mindestens einen Vernetzer, den Polymeren und dem Glycerin dynamisch gebrochen und wieder gebunden werden können. Das System bewegt sich dabei immer wieder in ein Gleichgewicht zurück. Auf Grund dieser Eigenschaft lässt sich die gelartige Knetmasse strecken und zusammendrücken aber auch reissen und wieder zusammenfügen. Ein FormGedächtnis aufgrund der Elastizität ist nur auf kurzfristige und schnelle Verformung beschränkt.

Aufgrund des 3-dimensionalen Netzwerks der gelartigen Knetmasse kann der Augentupfer mehrmals wiederverwendet werden, indem die "verbrauchte" Oberfläche der Masse nach innen gefaltet wird und sich dabei eine neue "unverbrauchte" Oberfläche bildet.

In einer weiteren Ausführungsform kann die gelartige Knetmasse zusätzlich Polymer-Mikrokugeln, vorzugsweise mit einem mittleren Partikelgrösse von 100 bis 150 Mikrometern, enthalten. Die Polymer-Mikrokugeln können expandierte Polymer-Mikrokugeln mit einer geringen Dichte von 30 bis 130 kg/m³, vorzugsweise 65 bis 100 kg/m³ sein. Diese können beispielsweise einen Kern aus Acrylnitril Copolymer mit einer inerten Beschichtung aus Kalziumkarbonat aufweisen (z.B. Dualite®, E065-135D, Henkel Cooperation, USA).

Expandierte Mikrokugeln sind besonders vorteilhaft, da sie zu einer besonders weichen, geschmeidigen und leichten gelartigen Knetmasse beitragen, welche den Komfort bei der Anwendung erhöhen und das Verletzungsrisiko der Horn- oder Lederhaut stark verringern.

Um das chemische Gleichgewicht des 3-dimensionalen Netzwerks zu stabilisieren, kann die gelartige Knetmasse zur Herstellung von Pufferlösungen geeignete Salze, insbesondere Natriumphosphate, enthalten. Der pH in der gelartigen Knetmasse ist dabei in der Regel in einem Bereich von 6.8 bis 8.0, vorzugsweise etwa 7.0 bis 7.6, eingestellt.

Das mindestens eine natürliche oder synthetische Polymer kann ausgewählt sein aus der Gruppe bestehend aus Galactomannan, insbesondere Hydroxypropyl Guar (HPG) oder Carboxymethyl Guar (CMG), Chitosan, polymerisiertes Siloxan und Kombinationen davon. Beispielsweise kann Chitosan durch deren Eignung als Mittel zum Binden oder Aufsaugen von Fetten, das Entfernen und Absorbieren von Verunreinigungen und Augenfluss aus den Augen unterstützen.

Das mindestens eine Polymer formt das eigentliche 3-dimensionale Gerüst, welches über reversible Vernetzer, z.B. Bor (als Borax oder Borsäure) oder die in PCT/EP2013/058101 beschriebenen amphiphilen Nanopartikeln, zusammengehalten werden. Glycerin, welches sich ebenfalls teilweise mit den anderen Komponenten vernetzen kann dient hauptsächlich der Wasserretention und der Einstellung der Klebrigkeit, so dass die Oberfläche des Augentupfers eine genügende Feuchtigkeit aufweist und bei Berührung mit empfindlichen Stellen des Auges nicht haftet. Zudem wird ein schnelles Austrocknen des Augentupfers verhindert.

Weiter kann die gelartige Knetmasse Propylen Glycol aufweisen, welches ebenfalls die Wasserretention erhöht und ein Austrocknen des Augentupfers verhindert.

Die Oberflächenfeuchte und die Klebrigkeit der gelartigen Knetmasse kann derart eingestellt sein, dass sie schädliche Partikel einfach und effizient absorbiert und dabei aus den Augen entfernt.

Um eine möglichst lange Anwendung zu gewährleisten, kann der Augentupfer bis zur Wiederverwendung in einem dicht verschliessbaren Behälter gelagert sein. In der Regel weist der Augentupfer ein Volumen von 10 bis 100 Milliliter, vorzugsweise 20 bis 50 Milliliter, auf.

In der gelartigen Knetmasse des Augentupfers können zur Augenpflege geeignete Wirkstoffen eingebettet sein.

Die gelartige Knetmasse kann weiter eine Wasserretention aufweisen, die derart gewählt ist, dass trotz Bildung von Oberflächenfeuchtigkeit ein zu starkes Austrocknen verhindert ist.

Weiter kann die gelartige Knetmasse Inhaltstoffe ausgewählt aus der Gruppe bestehend aus Konservierungsmitteln, Desinfektionsmitteln, Farbstoffen, Pigmenten, ätherischen Ölen oder Ölmischungen, Duftstoffen, und Kombinationen davon, enthalten.

In einer weiteren Ausführungsform des Augentupfers umfasst die gelartige Knetmasse als Grundzusammensetzung folgende Komponenten:

| | Menge (Gew.% - w/w) |
|---|---|
| Polymer (vorzugsweise Galactomannan und Chitosan) | 4.5 - 7.5 % |
| Bor (als Borax oder Borsäure) | 0.1 - 0.5% |
| Glycerin | 4.8 - 7.2 % |
| Propylene Glycol | 4.8 - 7.2 % |
| Polymer-Mikrokugeln | 2 - 4% |
| Weitere Inhaltstoffe | 1 - 2 % |
| Water | Rest (bis total 100 %) |

## Patentansprüche

1. Augentupfer zur Reinigung von Wirbeltieraugen, beispielsweise von Hundeaugen, **dadurch gekennzeichnet, dass** der Augentupfer aus einer gelartige Knetmasse besteht, wobei die gelartige Knetmasse aus einem reversibel gebundenen, 3-dimensionalen Netzwerk aus mindestens einem natürlichen oder synthetischen Polymer, mindestens einem Vernetzer und Glycerin zusammengehalten ist.

2. Augentupfer nach Anspruch 1, **dadurch gekennzeichnet, dass** die gelartige Knetmasse Polymer-Mikrokugeln enthält.

3. Augentupfer nach einem der vorangehenden Ansprüche, **dadurch gekennzeichnet, dass** die Polymer-Mikrokugeln eine mittlere Partikelgrösse von 100 bis 150 Mikrometern aufweisen.

4. Augentupfer nach einem der vorangehenden Ansprüche, **dadurch gekennzeichnet, dass** die Polymer-Mikrokugeln eine Dichte von 30 bis 130 kg/m³, vorzugsweise 65 bis 100 kg/m³ aufweisen.

5. Augentupfer nach einem der vorangehenden Ansprüche, **dadurch gekennzeichnet, dass** das mindestens eine natürliche oder synthetische Polymer ausgewählt ist aus der Gruppe bestehend aus Galactomannan, Chitosan, polymerisiertes Siloxan und Kombinationen davon.

6. Augentupfer nach einem der vorangehenden Ansprüche, **dadurch gekennzeichnet, dass** die gelartige Knetmasse eine Wasserretention aufweist, die derart gewählt ist, dass trotz Bildung von Oberflächenfeuchtigkeit ein zu starkes Austrocknen verhindert ist.

7. Augentupfer nach einem der vorangehenden Ansprüche, **dadurch gekennzeichnet, dass** zur Augenpflege geeignete Wirkstoffen in der gelartigen Knetmasse eingebettet sind.

8. Augentupfer nach einem der vorangehenden Ansprüche, **dadurch gekennzeichnet, dass** die gelartige Knetmasse weitere Inhaltstoffe ausgewählt aus der Gruppe bestehend aus Konservierungsmitteln, Desinfektionsmitteln, Farbstoffe, Pigmente, ätherische Öle oder Ölmischungen, Duftstoffen, und Kombinationen davon, enthält.

9. Augentupfer nach einem der vorangehenden Ansprüche, **dadurch gekennzeichnet, dass** die gelartige Knetmasse zur Herstellung von Pufferlösungen geeignete Salze, insbesondere Natriumphosphate, enthält.

10. Augentupfer nach einem der vorangehenden Ansprüche, **dadurch gekennzeichnet, dass** der pH in der gelartigen Knetmasse in einem Bereich von 6.8 bis 8.0, vorzugsweise etwa 7.0 bis 7.6, eingestellt ist.

11. Augentupfer nach einem der vorangehenden Ansprüche, **dadurch gekennzeichnet, dass** er ein Volumen von 10 bis 100 Milliliter, vorzugsweise 20 bis 50 Milliliter, aufweist.

12. Augentupfer nach einem der vorangehenden Ansprüche, **dadurch gekennzeichnet, dass** die gelartige Knetmasse als Grundzusammensetzung folgende Komponenten umfasst:
- Polymer 4.5 - 7.5 Gew.%
- Bor (als Borax oder Borsäure) 0.1 - 0.5 Gew.%
- Gylcerin 4.8 - 7.2 Gew.%
- Propylen Glycol 4.8 - 7.2 Gew.%
- Polymer-Mikrokugeln 2 - 4 Gew.%
- Weitere Inhaltsstoffe 1 - 2 Gew.%
und Wasser bis total 100 Gew.%.

13. Augentupfer nach Anspruch 12, **dadurch gekennzeichnet, dass** das Polymer Galactomannan und Chitosan umfasst.
